# EUROPÄISCHE PATENTANMELDUNG

(11) **EP 1 421 964 A2**
(43) Veröffentlichungstag der Anmeldung: **26.05.2004**
(21) Anmeldenummer: 03025627.5
(22) Anmeldetag: 06.11.2003
(51) Int. Cl.: A61M 5/32, A61M 5/50

(54) **Ampullenhalter**

(30) Priorität: 20.11.2002 DE 10254195; 05.12.2002 DE 20218864 U
(71) Anmelder: Pajunk OHG Besitzverwaltung, 78187 Geisingen (DE)
(72) Erfinder: Pajunk, Heinrich, 78187 Geisingen (DE); Pajunk, Horst, 78187 Geisingen (DE)
(74) Vertreter: Manitz, Finsterwald & Partner GbR

(57) **Zusammenfassung**

Es wird ein Ampullenhalter für Zylinderampullenspritzen mit einem einen innen liegenden Hohlraum (4) aufweisenden Grundkörper (1) beschrieben. In den Grundkörper (1) ist eine zu injizierende Flüssigkeit (30) enthaltene Ampulle (28) einsetzbar, wobei am proximalen Ende (19) des Grundkörpers (1) eine Eintrittsöffnung (9) ausgebildet ist, über die eine Kolbenstange (34) der Zylinderampullenspritze in den Hohlraum (4) einschiebbar ist. Am distalen Ende (18) des Grundkörpers (1) ist eine mit einer Injektionsnadel (39) koppelbare oder versehene Austrittsöffnung (5) ausgebildet. Die zu injizierende Flüssigkeit (30) ist bei einem Verschieben der Kolbenstange (34) aus der Ampulle (28) durch die Austrittsöffnung (5) oder die Injektionsnadel (39) herauspressbar. An der Außenseite des Grundkörpers (1) ist eine Schutzhülle (16,55) vorgesehen, die von einer zurückgezogenen Ruheposition, in der die Injektionsnadel (39) im Wesentliche über ihre gesamte Länge freiliegt, in eine vorgeschobene Sicherungsposition verschiebbar, in der die Injektionsnadel (39) über ihre gesamte Länge innerhalb der Schutzhülle (16,55) angeordnet ist.

## Beschreibung

Die vorliegende Erfindung betrifft einen Ampullenhalter für Zylinderampullenspritzen mit einem einen innen liegenden Hohlraum aufweisenden Grundkörper, in den eine eine zu injizierende Flüssigkeit enthaltende Ampulle einsetzbar ist, wobei am proximalen Ende des Grundkörpers eine Eintrittsöffnung ausgebildet ist, über die eine Kolbenstange der Zylinderampullenspritze in den Hohlraum einschiebbar ist, am distalen Ende des Grundkörpers eine mit einer Injektionsnadel koppelbare oder versehene Austrittsöffnung ausgebildet ist, und die zu injizierende Flüssigkeit bei einem Verschieben der Kolbenstange aus der Ampulle durch die Austrittsöffnung oder die Injektionsnadel herauspressbar ist.

Ampullenhalter dieser Art werden beispielsweise in der Dentalmedizin zur Injektion von schmerzstillenden Mitteln verwendet. Bei einem bekannten Spritzensystem ist der Ampullenhalter mit seinem proximalen Ende schwenkbar an dem Spritzenkörper gelagert. Bei einem Verschwenken des Ampullenhalters wird die Eintrittsöffnung freigegeben, so dass die Zylinderampulle durch die Eintrittsöffnung in den Ampullenhalter eingeschoben werden kann. Nach vollständigem Zurückschwenken des Ampullenhalters wird dieser automatisch fixiert. Nach Vorschieben der Kolbenstange kann diese durch eine am distalen Ende der Kolbenstange vorgesehene Klemmvorrichtung mit einem am proximalen Ende der Zylinderampulle vorgesehenen Kolben gekoppelt werden, so dass ein Vor- und Zurückschieben des Kolbens über die Kolbenstange möglich ist. Nach dieser Vorbereitung wird auf die am distalen Ende des Ampullenhalters vorgesehene Austrittsöffnung eine Injektionsnadel aufgeschraubt, welche zur Vermeidung von Verletzungen mit einer Schutzkappe gesichert ist.

Bei einer unter der Bezeichnung "Variosystem" bekannten Abwandlung ist der Ampullenhalter als separates Teil ausgebildet, das vollständig von der restlichen Injektionsspritze, dem Griffstück der Injektionsspritze, abnehmbar ist. Der Ampullenhalter besitzt an seinem proximalen Ende einen ringförmigen Ansatz, mit der er in eine entsprechende Aufnahme am Griffstück eingesetzt und fixiert werden kann.

Das Einsetzen der Zylinderampulle in den Ampullenhalter erfolgt bei diesem System bei abgenommenem Ampullenhalter. Nachdem die Ampulle durch die proximale Eintrittsöffnung in den Ampullenhalter eingeführt ist, wird der Ampullenhalter an das Griffstück der Injektionsspritze angekoppelt. Die übrige Vorbereitung, d. h. das Ankoppeln der Kolbenstange an die Zylinderampulle sowie das Aufschrauben der Injektionsnadel erfolgt in der bereits beschriebenen Weise.

Beiden Systemen ist gemeinsam, dass die gesamte Injektionsspritze, d.h. der Ampullenhalter und das Griffstück, als mehrfach zu verwendendes Instrumententeil üblicherweise aus Metall ausgebildet ist. Somit ist nach durchgeführter Injektion die gesamte Injektionsspritze, einschließlich des Ampullenhalters, einer Sterilisation zuzuführen, die mit Kosten verbunden ist.

Weiterhin sind in den bekannten Ampullenhaltern seitlich jeweils zwei lang gestreckte Öffnungen vorgesehen, über die zum einen ein bei einer vor der Injektion durchgeführten Aspiration entstehender Blutfaden beobachtet werden kann und zum anderen während der Injektion die jeweilige Position des Kolbens ersichtlich ist. Weiterhin kann nach der durchgeführten Injektion die Zylinderampulle durch diese seitlichen Öffnungen gefasst und durch die Eintrittsöffnungen axial aus dem Ampullenhalter herausgezogen werden.

Da im Dentalbereich Injektionsnadeln mit sehr geringen Durchmessern verwendet werden, sind die bei der Injektion aufzuwendenden Druckwerte sehr hoch. In seltenen Fällen kann dieser hohe Druck dazu führen, dass die sich in dem Ampullenhalter befindende Zylinderampulle während der Injektion platzt, so dass die Glassplitter der geplatzten Ampulle zusammen mit dem noch in der Ampulle befindlichen Injektionsmittel direkt in den Mund des Patienten fallen bzw. fließen können.

Die verwendeten Injektionskanülen bestehen zum einen üblicherweise aus der für die eigentliche Injektion verwendeten Injektionsnadel (Hohlnadel mit Spitze), die proximalseitig ein aus Kunststoff bestehendes Kopplungselement zur Befestigung an der Austrittsöffnung des Ampullenhalters aufweist. Zum anderen erstreckt sich die Injektionskanüle proximalseitig durch das Kopplungselement hindurch und endet in einem rückwärtigen Kanülenabschnitt, der in den Ampullenhalter und die darin angeordnete Zylinderampulle hineinragt. Im Rahmen dieser Anmeldung wird der Begriff Injektionsnadel für den außen liegenden, für die eigentliche Injektion verwendeten Teil und der Begriff rückwärtiger Kanülenabschnitt für den in das Innere des Ampullenhalters hineinragende Teil verwendet.

Das Hauptproblem bei den bekannten Injektionsspritzen liegt jedoch in der Verletzungsgefahr durch die Injektionsnadel. Dabei ist das Verletzungsrisiko durch eine Injektionsnadel insbesondere nach durchgeführter Injektion zu berücksichtigen, da ab diesem Zeitpunkt die Injektionsnadel als mikrobiell kontaminiert angesehen werden muss. Abhängig von einer eventuellen Erkrankung des Patienten (beispielsweise HIV oder Hepatitis) kann eine Verletzung mit einer derartig kontaminierten Injektionsnadel erhebliche Risiken mit sich bringen.

Die Verletzungsgefahr beginnt dabei unmittelbar nach der Injektion, d. h. bereits mit der Übergabe der Injektionsspritze vom Arzt an die Arzthelferin. Auch beim weiteren Transport der Injektionsspritze vom Behandlungsraum zum Sterilisationsraum sind Verletzungen durch die Injektionsnadel möglich.

Um entsprechende Verletzungen zu vermeiden, wurde in der Vergangenheit beispielsweise ein so genanntes "Recapping" durchgeführt, d. h. nach erfolgter Injektion wurde die ursprüngliche Schutzkappe wieder auf die Injektionsnadel aufgesetzt. Gerade beim Aufsetzen der Schutzkappe auf die Injektionsnadel ist jedoch die Gefahr einer Stichverletzung vorhanden, so dass beispielsweise in den USA das "Recapping" verboten wurde.

In der Vergangenheit wurde eine Vielzahl von Vorrichtungen vorgestellt, mit denen nach durchgeführter Injektion die Injektionsnadel von dem Ampullenhalter gefahrlos abgeschraubt und gleichzeitig entsorgt werden kann. Diese oftmals elektrisch betriebenen Vorrichtungen sind jedoch relativ kostspielig, so dass in den meisten Fällen solche Apparate nicht in jedem Behandlungszimmer, sondern bestenfalls nur im Sterilisationsraum vorhanden sind. Die Gefahr einer Verletzung auf dem Transportweg vom Behandlungsraum zum Sterilisationsraum wird daher durch diese Vorrichtungen nicht ausgeschlossen.

Es ist eine Aufgabe der vorliegenden Erfindung, einen Ampullenhalter der eingangs genannten Art so auszubilden, dass eine Verletzung durch eine kontaminierte Injektionsnadel praktisch ausgeschlossen werden kann.

Diese Aufgabe wird erfindungsgemäß ausgehend von einem Ampullenhalter der eingangs genannten Art dadurch gelöst, dass an der Außenseite des Grundkörpers eine Schutzhülle vorgesehen ist, die von einer zurückgezogenen Ruheposition, in der die Injektionsnadel im Wesentlichen über ihre gesamte Länge frei liegt, in eine vorgeschobene Sicherungsposition verschiebbar ist, in der die Injektionsnadel über ihre gesamte Länge innerhalb der Schutzhülle angeordnet ist.

Im Rahmen dieser Anmeldung wird der Begriff "proximal" mit der Bedeutung "zum Körper des Arztes hin gelegen" verwendet. Dementsprechend wird der Begriff "distal" mit der Bedeutung "vom Körper des Arztes entfernt gelegen" verwendet.

Durch die Erfindung wird erreicht, dass Stichverletzungen nach durchgeführter Injektion praktisch ausgeschlossen sind, wobei gleichzeitig die Handhabung der Injektionsspritze vor und während der Injektion in unveränderter Weise beibehalten werden kann. Dadurch, dass die Schutzhülle in der zurückgezogenen Ruheposition die Injektionsnadel über ihre gesamte Länge freigibt, ist die Handhabung der mit dem erfindungsgemäßen Ampullenhalter versehenen Injektionsspritze gegenüber den bisherigen Systemen völlig unverändert.

Die Sicherung der Injektionsnadel ist mit dem erfindungsgemäß ausgebildeten Ampullenhalter so einfach möglich, dass sie direkt von dem Arzt unmittelbar nach Beendigung der Injektion durchgeführt werden kann. Für die Sicherung muss vom Arzt lediglich nach Beendigung der Injektion die Schutzhülle von der zurückgezogenen Ruheposition in die vorgeschobene Sicherungsposition vorgeschoben werden, in der die Injektionsnadel über ihre gesamte Länge von der Schutzhülle verdeckt ist. Somit wird durch die Erfindung bereits zum frühest möglichen Zeitpunkt, d. h. unmittelbar nach der durchgeführten Injektion, eine Sicherung der Injektionsnadel gewährleistet, so dass Stichverletzungen bei korrekter Handhabung ausgeschlossen sind. Da die Sicherung der Injektionsnadel bereits vom behandelnden Arzt durchgeführt wird, kann selbst bei der Übergabe der Injektionsspritze vom Arzt zur Arzthelferin keine Stichverletzung mehr auftreten. Die Wirkung der Erfindung beginnt somit bereits zu einem wesentlich früheren Zeitpunkt als alle bekannten sonstigen Sicherheitssysteme, wie sie eingangs beschrieben wurden.

Da die Schutzhülle, im Gegensatz zu der Bewegung beim "Recapping", nicht auf die freie Spitze der Injektionsnadel aufgesetzt, sondern ausgehend von der Basis der Nadel in Richtung zur Nadelspitze verschoben wird und gleichzeitig die Schutzhülle und die Injektionsnadel durch ihre gemeinsame Festlegung an dem Grundkörper zu jedem Zeitpunkt eine eindeutige und fest vorgegebene Position zueinander besitzen, ist es ausgeschlossen, dass beim Vorschieben der Schutzhülle die Spitze der Injektionsnadel berührt wird.

Nach einer vorteilhaften Ausführungsform der Erfindung ist die Injektiorisnadel zumindest im Bereich ihrer Spitze bei in der Sicherungsposition befindlicher Schutzhülle von dieser seitlich vollständig umschlossen. Grundsätzlich ist es ausreichend, wenn in der Sicherungsposition zumindest die Spitze der Injektionsnadel seitlich abgedeckt ist. Bevorzugt ist die Injektionsnadel jedoch über ihre gesamte Länge bei in der Sicherungsposition befindlicher Schutzhülle von dieser seitlich vollständig umschlossen. Bei dieser Ausbildung ist sichergestellt, dass die Injektionsnadel auch unterhalb der Spitze nicht berührt werden kann, so dass eine Kontamination vollständig ausgeschlossen ist.

Bevorzugt ragt das distale Ende der sich in der Sicherungsposition befindenden Schutzhülle über die Spitze der Injektionsnadel hinaus. Das distale Ende der Schutzhülle ist dabei bevorzugt so weit nach vorne zu schieben, dass ein Berühren der Spitze der Injektionsnadel auch mit der Fingerspitze ausgeschlossen ist. Auf diese Weise ist sichergestellt, dass auch beim Berühren des distalen Endes der vorgeschobenen Schutzhülle, beispielsweise mit der Fingerspitze, eine Stichverletzung ausgeschlossen ist.

Nach einer weiteren vorteilhaften Ausführungsform der Erfindung ist die Schutzhülle in der Sicherungsposition gegenüber dem Grundkörper unverschiebbar fixierbar. Auf diese Weise wird sichergestellt, dass beispielsweise auch bei einem Schlag auf das vorgeschobene distale Ende der Schutzhülle diese nicht in die Ruheposition zurückgeschoben wird und die Spitze der Injektionsnadel dadurch wieder aus der Schutzhülle austreten könnte.

Bevorzugt erfolgt bei Erreichen der Sicherungsposition eine automatische Fixierung der Schutzhülle. Diese automatische Fixierung gewährleistet beim vollständigen Vorschieben der Schutzhülle eine Zwangsfixierung, so dass die Fixierung im Rahmen einer Fehlbedienung nicht vergessen werden kann.

Nach einer weiteren bevorzugten Ausführungsform der Erfindung ist zur Fixierung der Schutzhülle an dieser zumindest ein Rastelement vorgesehen, das in der Sicherungsposition mit zumindest einem an dem Grundkörper vorgesehenen ersten Gegenelement zur Fixierung der Schutzhülle zusammenwirkt. Über ein Rastelement ist eine sehr einfache und kostengünstige Fixierung der Schutzhülle gegenüber dem Grundkörper möglich. Dabei müssen das Rastelement und das mit diesem zusammenwirkende erste Gegenelement so ausgebildet sein, dass nach erfolgter Verrastung ein Zurückschieben der Schutzhülle nicht mehr möglich ist. Dazu kann das Rastelement beispielsweise als Rast-Schnapp-Element ausgebildet sein.

Grundsätzlich kann das Rastelement als separates Teil oder einstückig mit der Schutzhülle ausgebildet sein.

Nach einer weiteren vorteilhaften Ausführungsform der Erfindung umfasst das Rastelement eine Rastnase, während das erste Gegenelement als Rastausnehmung ausgebildet ist, in die die Rastnase in der Sicherungsposition eingreift. Bevorzugt sind dabei das Rastelement im Bereich des proximalen Endes der Schutzhülle und das erste Gegenelement im Bereich des distalen Endes des Grundkörpers ausgebildet. Grundsätzlich ist es auch möglich, dass Rastnase und Rastausnehmung gegeneinander vertauscht sind, d. h. dass die Rastnase an dem Grundkörper und die Rastausnehmung an der Schutzhülle vorgesehen sind.

Nach einer weiteren vorteilhaften Ausführungsform der Erfindung umfasst das Rastelement einen C-förmigen Basisteil, an dessen radialer Innenseite eine oder mehrere, bevorzugt zwei sich radial nach innen erstreckende Rastnasen ausgebildet sind. Bevorzugt sind zwei Rastnasen vorgesehen, die sich einander gegenüberliegend angeordnet sind.

Insbesondere wird die Schutzhülle von dem C-förmigen Basisteil radial umgriffen, wobei bevorzugt in der außen liegenden Oberfläche der Schutzhülle eine ringförmige Vertiefung, insbesondere eine Ringnut vorgesehen ist, in der der C-förmige Basisteil angeordnet ist. Weiterhin ist vorteilhaft in der Schutzhülle für jede Rastnase eine Öffnung vorgesehen, durch die sich die jeweilige Rastnase hindurch in Richtung zur Außenseite des Grundkörpers hin erstreckt.

Grundsätzlich ist es auch möglich, dass der C-förmige Basisteil unmittelbar das Rastelement bildet. In diesem Fall könnte der C-förmige Basisteil das proximale Ende der Schutzhülle so in axialer Richtung umgreifen, dass ein über das proximale Ende der Schutzhülle hinaus ragender Teil des C-förmigen Basisteiles an der Außenseite des Grundkörpers zur Anlage kommt.

Vorteilhaft liegen die Rastelemente unter Vorspannung an der Außenseite des Grundkörpers an. Dadurch ist gewährleistet, dass die Rastelemente automatisch nach vollständigem Vorschieben der Schutzhülle in die entsprechenden ersten Gegenelemente eingreifen und somit die gewünschte Zwangsfixierung bewirken.

Nach einer weiteren vorteilhaften Ausführungsform der Erfindung sind an der Außenseite des Grundkörpers eine oder mehrere Längsführungen für die Schutzhülle vorgesehen. Die Längsführungen können dabei als Längsstege ausgebildet sein, so dass durch die Längsstege gleichzeitig eine Verstärkung des Grundkörpers erfolgt. In den Rastelementen können in diesem Fall Führungsnuten ausgebildet sein, die beim Verschieben der Schutzhülle auf den Längsstegen geführt werden.

Grundsätzlich ist es auch möglich, dass die Längsführungen als Längsnuten ausgebildet sind, wobei in diesem Fall die Rastelemente beim Verschieben der Schutzhülle in den Längsnuten geführt werden.

Die Längsführungen bewirken eine Verdrehsicherung zwischen Schutzhülle und Grundkörper, so dass verhindert wird, dass die unter Vorspannung an der Außenseite des Grundkörpers anliegenden Rastelemente bei einem Verdrehen der Schutzhülle in an der Außenseite des Grundkörpers eventuell vorhandene Vertiefungen eingreifen und ein Verschieben der Schutzhülle stören würden.

Bevorzugt sind die Schutzhülle und der Grundkörper gegeneinander unverdrehbar ausgebildet. Diese Unverdrehbarkeit kann dabei durch die bereits genannten Längsführungen, aber auch durch sonstige geeignete Mittel realisiert sein. Beispielsweise kann der Außenquerschnitt des Grundkörpers und der Innenquerschnitt der Schutzhülle von der konzentrischen Form abweichen, insbesondere eine ovale, eckige oder sonstige geeignete Form besitzen, durch die eine Verdrehsicherheit gegeben ist.

Nach einer weiteren vorteilhaften Ausführungsform der Erfindung ist die Schutzhülle in der Ruheposition mit dem Grundkörper unverschiebbar gekoppelt, wobei diese Koppelung lösbar ausgebildet ist. Bevorzugt handelt es sich bei dieser lösbaren Koppelung um eine lösbare Verrastung. Durch diese lösbare Koppelung ist sichergestellt, dass die Schutzhülle nicht versehentlich zu einem falschen Zeitpunkt, beispielsweise vor oder während der Injektion, von der Ruheposition in die Sicherungsposition überführt wird.

Nach einer vorteilhaften Ausführungsform der Erfindung erfolgt die lösbare Verrastung in der Ruheposition über das Rastelement und ein im proximalen Bereich des Grundkörpers vorgesehenes zweites Gegenelement. Durch die doppelte Verwendung des Rastelementes, einmal zur lösbaren Verrastung in der Ruheposition und zum anderen zur der nicht lösbaren Verrastung in der Sicherungsposition ist eine Kostenreduzierung beim Herstellen des erfindungsgemäßen Ampullenhalters möglich.

Nach einer weiteren vorteilhaften Ausführungsform der Erfindung weist der Grundkörper eine oder mehrere, bevorzugt zwei seitliche Öffnungen auf, durch die der innen liegende Hohlraum von außen zugänglich ist. Über diese seitlichen Öffnungen kann nach erfolgter Injektion und nach dem Vorschieben der Schutzhülle die Ampulle von außen ergriffen und nach hinten aus dem Grundkörper heraus geschoben werden. Dazu erstrecken sich die seitlichen Öffnungen bevorzugt in Richtung der Längsachse des Grundkörpers, um auf diese Weise das Entnehmen der Ampulle zu erleichtern.

Nach einer weiteren bevorzugten Ausführungsform der Erfindung werden die seitlichen Öffnungen bei sich in der Ruheposition befindender Schutzhülle vollständig von dieser verschlossen. Falls während der Injektion aufgrund des aufgewandten hohen Druckes die sich im Inneren des Grundkörpers befindende Ampulle platzen sollte, so wird durch diese Ausbildung gewährleistet, dass keine Splitter und keine Injektionsflüssigkeit durch die seitlichen Öffnungen im Grundkörper austreten und beispielsweise in den Mund des Patienten fallen bzw. fließen können.

Nach einer weiteren bevorzugten Ausführungsform ragt das distale Ende der Schutzhülle über das distale Ende des Grundkörpers, insbesondere über die Austrittsöffnung, hinaus, wenn sich die Schutzhülle in der Ruheposition befindet. Dadurch kann verhindert werden, dass die Austrittsöffnung berührt und somit kontaminiert wird. Bei einer kontaminierten Austrittsöffnung könnten Partikel in die zu injizierende Flüssigkeit gelangen und den Patienten infizieren.

Nach einer weiteren vorteilhaften Ausführungsform der Erfindung erstreckt sich im Inneren des Grundkörpers ausgehend von der Austrittsöffnung ein Ansatz in den Hohlraum hinein. Bevorzugt besitzt das sich im Inneren des Hohlraums hin erstreckende freie Ende des Ansatzes eine geringere Außenabmessung, insbesondere einen geringen Außendurchmesser, als eine am distalen Ende einer in den Hohlraum einzusetzenden Ampulle vorgesehene, üblicherweise mit einer Membran verschlossene Öffnung.

Durch diesen Ansatz wird bei vollständig in den Hohlraum eingesetzter Ampulle und Vorschieben der Kolbenstange die Membran durch den Ansatz zum Inneren der Ampulle hin gedrückt, was zu einer Zwangsaspiration führt. Da die verwendeten Injektionsampullen üblicherweise an ihrem distalen Ende mit einer elastischen Membran, beispielsweise einer Gummimembran, verschlossen sind, durch die der rückwärtige Kanülenabschnitt der Injektionskanüle hindurch gestochen wird, kann die erfindungsgemäße Zwangsaspiration durch folgende Vorgehensweise erreicht werden. Zunächst wird in üblicher Weise der zu betäubende Bereich des Zahnfleisches mit der Injektionsnadel punktiert. Anschließend wird der Kolben der Injektionsampulle durch ein geringfügiges Vorschieben der Kolbenstange in bekannter Weise in distaler Richtung bewegt, woraufhin anschließend der Druck auf die Kolbenstange wieder beendet wird. Beim Vorschieben der Kolbenstange wurde die elastische Membran durch den Ansatz elastisch verformt, so dass bei Nachlassen des Druckes die Membran wieder in ihre Ruheposition zurückgeht. Durch diese Bewegung entsteht innerhalb der Injektionsampulle ein Unterdruck, so dass eine Zwangsaspiration erfolgt, ohne dass der Arzt die Kolbenstange aktiv zurückziehen muss.

Nach einer weiteren vorteilhaften Ausführungsform der Erfindung bestehen der Grundkörper und/ oder die Schutzhülle aus Kunststoff. Auf diese Weise ist eine sehr kostengünstige Herstellung des erfindungsgemäß ausgebildeten Ampullenhalters möglich. Insbesondere ist es vorteilhaft, wenn der erfindungsgemäß ausgebildete Ampullenhalter als Einmalprodukt hergestellt ist, da auf diese Weise eine Sterilisation nicht erforderlich ist. Der erfindungsgemäße Ampullenhalter kann in diesem Fall nach durchgeführter Injektion zusammen mit der sich innerhalb der vorgeschobenen Schutzhülle befindenden Injektionsnadel entsorgt werden. Bevorzugt sind die Schutzhülle sowie der Grundkörper dabei als Spritzgusselemente herstellbar.

Da das vergleichsweise teure Griffstück leicht gereinigt und mehrfach verwendet werden kann, können, um Kosten zu sparen, bei einer vorteilhaften Ausführungsform der Ampullenhalter und die Schutzhülle als Einmalprodukte ausgebildet sein, das Griffstück jedoch als Mehrwegprodukt.

Nach einer weiteren vorteilhaften Ausführungsform der Erfindung bestehen der Grundkörper und/oder die Schutzhülle aus transparentem oder durchsichtigem Material. Auf diese Weise ist gewährleistet, dass während der Injektion zum einen jeweils die Position des Ampullenkolbens beobachtet werden kann und zum anderen bei der Aspiration ein eventuell auftretender Blutfaden sicher erkannt wird.

Grundsätzlich kann die Injektionsnadel als vom Grundkörper getrenntes Teil ausgebildet sein, das mit der Austrittsöffnung koppelbar ist. Dabei ist bevorzugt im Bereich des proximalen Endes der Injektionsnadel ein Kopplungselement ausgebildet, über das die Injektionsnadel an der Austrittsöffnung ankoppelbar ist.

Bei dieser Ausführungsform sind somit handelsübliche separate Injektionskanülen verwendbar, die in üblicher Weise auf das distale Ende des Grundkörpers aufgesetzt, beispielsweise aufgeschraubt werden.

Nach einer weiteren vorteilhaften Ausführungsform der Erfindung kann die Injektionsnadel in den Grundkörper integriert, insbesondere mit dem Grundkörper verspritzt oder verklebt sein. Dazu ist bevorzugt zur Aufnahme der Injektionsnadel im Bereich des distalen Endes des Grundkörpers ein insbesondere konusförmiger Ansatz ausgebildet, durch den sich die Austrittsöffnung hindurch erstreckt. Der Ansatz besitzt dabei eine axiale Ausdehnung, die eine sichere Führung und Befestigung der Injektionskanüle gewährleistet.

Da der erfindungsgemäß ausgebildete Ampullenhalter im Gegensatz zu den bekannten Ampullenhaltern aus dem Stand der Technik vorteilhaft als Einmalprodukt hergestellt wird, ist eine Integration der Injektionsnadel bzw. der gesamten Injektionskanüle, die ebenfalls ein Einmalprodukt ist, in den Ampullenhalter möglich. Durch die integrierte Ausbildung entfällt somit die Notwendigkeit, die Injektionskanüle auf den Ampullenhalter aufzuschrauben, so dass die Anzahl der erforderlichen Schritte bei der Vorbereitung der Injektion verringert wird. Das erfindungsgemäße System ist in dieser Ausführungsform somit noch einfacher zu verwenden.

Bei einer bevorzugten Ausführungsform ist zwischen der Ruheposition und der Sicherungsposition der Schutzhülle eine Zwischensicherungsposition vorgesehen, in der die Schutzhülle gegen Verschieben gesichert ist, wobei diese Sicherung lösbar ausgebildet ist. Bevorzugt handelt es sich bei der lösbaren Sicherung um eine lösbare Verrastung. Dies hat den Vorteil, dass die Schutzhülle verschoben werden kann, um beispielsweise während einer Behandlung eine Ampulle auszutauschen oder während einer längeren Behandlung die Spritze abzulegen, ohne dass die Gefahr besteht, dass die Schutzhülle ungewollt in die Sicherungsposition gelangt. Nach dem Wechsel der Ampulle oder wenn die Spritze wieder benötigt wird, kann die Schutzhülle wieder in die Ruheposition geschoben werden, um die Behandlung fortzusetzen.

Bei einer vorteilhaften Ausführungsform ist die Injektionsnadel über ihre gesamte Länge innerhalb der Schutzhülle angeordnet, wenn sich diese in der Zwischensicherungsposition befindet. Somit können beispielsweise während des Wechselns der Ampulle oder des Ablegens der Spritze Stichverletzungen durch eine möglicherweise bereits kontaminierte Nadelspitze vermieden werden.

Bei einer weiteren bevorzugten Ausführungsform der Erfindung ist die lösbare Verrastung der Schutzhülle in der Zwischensicherungsposition so ausgebildet, dass das Rastelement mit zumindest einem im distalen Bereich des Grundkörpers vorgesehenen dritten Gegenelement zusammenwirkt. Da das Rastelement auch zur lösbaren Verrastung in der Ruheposition und zur nicht lösbaren Verrastung in der Sicherungsposition verwendet werden kann, ist eine einfache und kostengünstige Fixierung der Schutzhülle in der Zwischensicherungsposition möglich.

Bei einer vorteilhaften Ausführungsform ist am Grundkörper und/oder an der Schutzhülle eine Markierung zum Anzeigen der Sicherungsposition bzw. der Zwischensicherungsposition vorgesehen, so dass der Anwender optisch einfach erkennen kann, ob sich die Schutzhülle in der Sicherungsposition bzw. der Zwischensicherungsposition befindet.

Weitere vorteilhafte Ausführungsformen der Erfindung sind in den Unteransprüchen angegeben.

Die Erfindung wird nachfolgend anhand eines Ausführungsbeispiels unter Bezugnahme auf die Zeichnungen näher beschrieben; in diesen zeigen:
- Fig. 1: eine Seitenansicht auf den Grundkörper eines erfindungsgemäß ausgebildeten Ampullenhalters,
- Fig. 2: eine weitere Seitenansicht auf den Grundkörper nach Fig. 1,
- Fig. 3: einen Schnitt gemäß den Linien A-A nach Fig. 2,
- Fig. 4: einen Längsschnitt durch den Ampullenhalter gemäß Fig. 2,
- Fig. 5: eine Seitenansicht einer erfindungsgemäß ausgebildeten Schutzhülle,
- Fig. 6: eine Ansicht auf die distale Stirnseite der Schutzhülle nach Fig. 5,
- Fig. 7: einen Längsschnitt durch die Schutzhülle nach Fig. 5,
- Fig. 8: den Längsschnitt gemäß Fig. 4 mit eingesetzter Zylinderampulle,
- Fig. 9: einen Teillängsschnitt durch einen erfindungsgemäß ausgebildeten Ampullenhalter,
- Fig. 10: einen Querschnitt entlang der Linie B-B aus Fig. 9,
- Fig. 11: einen erfindungsgemäß ausgebildeten Ampullenhalter mit Injektionsnadel und Griffstück im entkoppelten Zustand,
- Fig. 12: eine Injektionsspritze mit erfindungsgemäß ausgebildetem Ampullenhalter, bei dem sich die Schutzhülle in der Ruheposition befindet,
- Fig. 13: die Vorrichtung nach Fig. 12, bei der sich die Schutzhülle in der vorgeschobenen Sicherungsposition befindet,
- Fig. 14: einen Teillängsschnitt durch die Vorrichtung nach Fig. 13,
- Fig. 15: einen Teillängsschnitt durch eine weitere Ausführungsform der Erfindung,
- Fig. 16: einen Längsschnitt durch eine weitere erfindungsgemäß ausgebildete Schutzhülle,
- Fig. 17: einen Teillängsschnitt durch eine Weiterbildung einer erfindungsgemäßen Ausführungsform des Ampullenhalters,
- Fig. 18: eine Teilseitenansicht eines erfindungsgemäßen Grundkörpers und einer erfindungsgemäßen Schutzhülle, wobei sich die Schutzhülle in der Zwischensicherungsposition befindet, und
- Fig. 19: eine Teilseitenansicht wie in Fig. 18, wobei sich die Schutzhülle in der Sicherungsposition befindet.

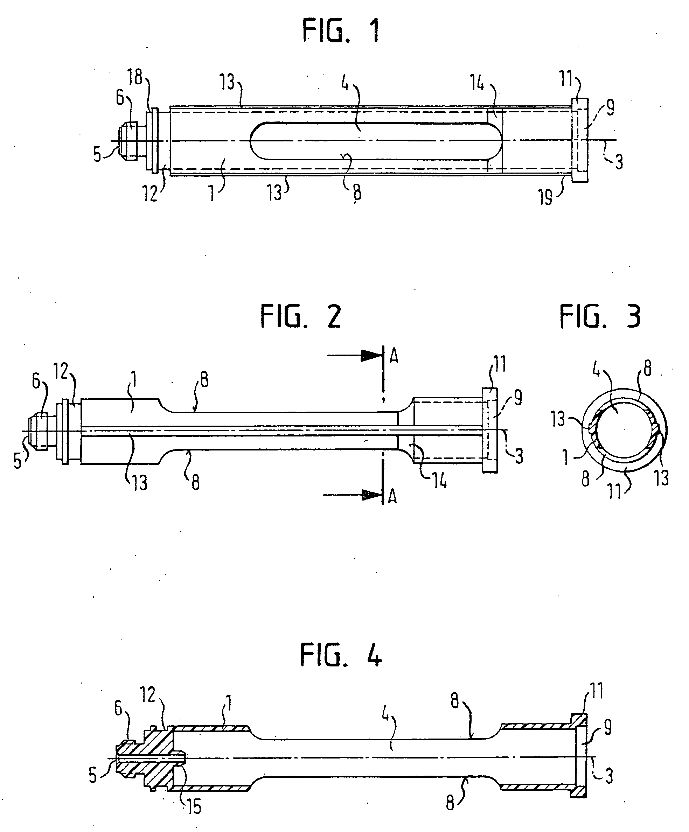

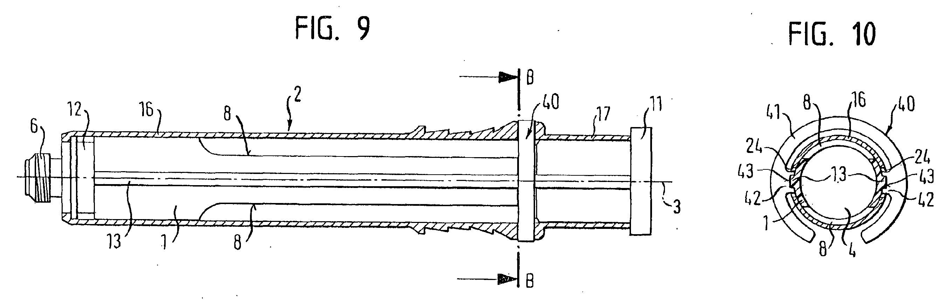

Fig. 1 zeigt einen Grundkörper 1 eines erfindungsgemäß ausgebildeten Ampullenhalters 2 (Fig. 9). Der Grundkörper 1 besitzt einen zylindrischen Aufbau mit einer Längsachse 3 und einem im Inneren ausgebildeten Hohlraum 4.

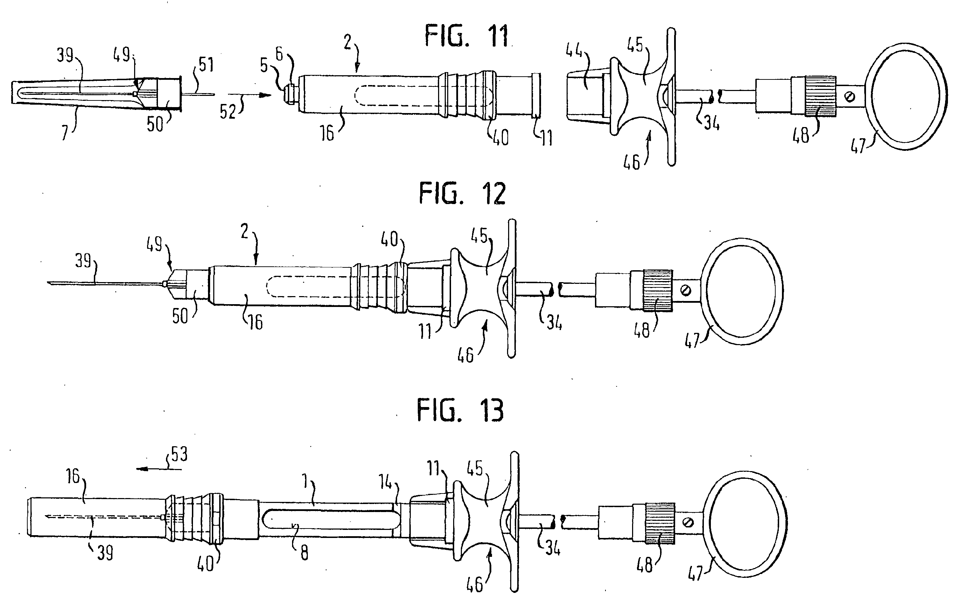

Der Hohlraum 4 endet an seinem distalen Ende in einer Austrittsöffnung 5, die, wie in Fig. 4 zu erkennen ist, als zur Längsachse 3 konzentrisch angeordneter Austrittskanal ausgebildet ist. Weiterhin ist im Bereich der Austrittsöffnung 5 eine außenseitig gelegene Koppelungsvorrichtung 6 vorgesehen, an der eine übliche Injektionsnadel 39 (siehe Figuren 11 bis 14) in üblicher Weise ankoppelbar, beispielsweise anschraubbar ist.

An zwei Längsseiten des Grundkörpers 1 sind jeweils lang gestreckte, ovale seitliche Öffnungen 8 ausgebildet, über die der Hohlraum 4 von außen zugänglich ist.

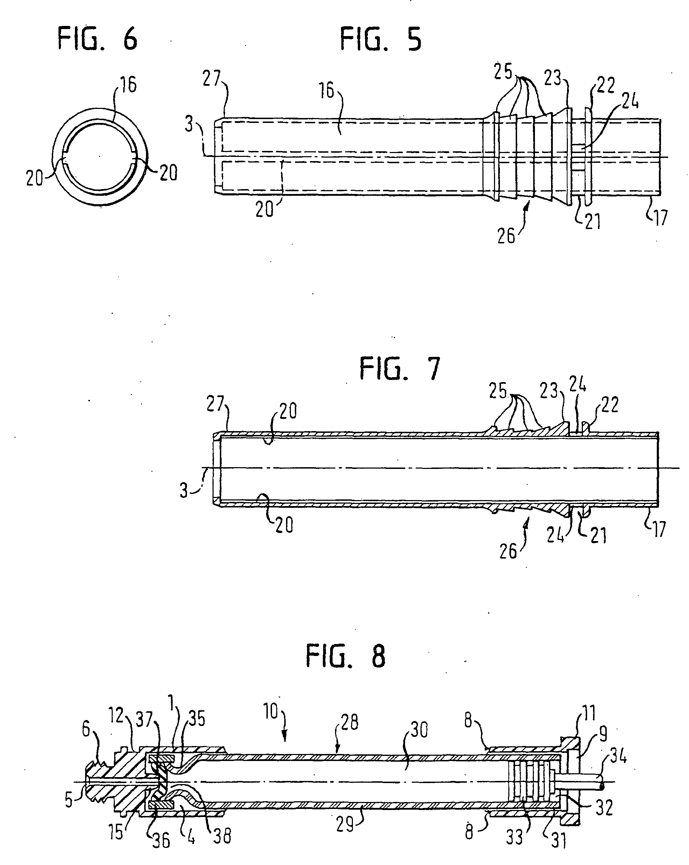

Weiterhin ist der Hohlraum 4 am proximalen Ende 19 des Grundkörpers 1 über eine Eintrittsöffnung 9 von außen zugänglich. Über diese Eintrittsöffnung 9 ist eine handelsübliche Zylinderampulle 10 (Fig. 8) in den Hohlraum 4 des Grundkörpers 1 einsetzbar. Weiterhin wird die Eintrittsöffnung 9 an dem proximalen Ende 19 des Grundkörpers 1 von einem ringförmigen Ansatz 11 umgeben.

Der bisher beschriebene Aufbau des Grundkörpers 1 entspricht im Wesentlichen einem Aufbau eines bekannten Ampullenhalters, wie er bei Injektionsspritzen nach dem bekannten Vario-System verwendet wird.

Im Gegensatz zu diesen bekannten Ampullenhaltern besitzt der erfindungsgemäß ausgebildete Grundkörper 1 im Bereich seines distalen Endes 18 eine umlaufenden Ringnut 12 sowie zwei sich entlang der Außenfläche des Grundkörpers 1 in Längsrichtung erstreckende Längsstege 13. Weiterhin ist eine weitere Ringnut 14 im Bereich des proximalen Endes des Grundkörpers 1 vorgesehen, wobei diese Ringnut 14 aufgrund der vorhandenen seitlichen Öffnungen 8 nicht vollständig umlaufend ausgebildet ist. Die Ringnut 12 besitzt darüber hinaus eine wesentlich größere Tiefe als die Ringnut 14. Weiterhin sind die bekannten Ampullenhalter als Mehrwegprodukte aus Metall ausgebildet, während der Grundkörper 1 gemäß der Erfindung bevorzugt als Einmalprodukt aus Kunststoff besteht.

Insbesondere aus den Figuren 2 und 3 ist nochmals die Ausbildung der Längsstege 13 deutlicher zu erkennen. Aus Fig. 3 ist dabei zu erkennen, dass sich die Längsstege 13 radial gegenüberliegen.

Aus Fig. 4 ist zu erkennen, dass an der innen liegenden Seite der Austrittsöffnung 5 ein ringförmiger Ansatz 15 ausgebildet ist, der sich in den Hohlraum 4 hinein erstreckt und konzentrisch um die Austrittsöffnung 5 herum verläuft. Die Außenseiten des Ansatzes 15 sind dabei konisch in Richtung zum Inneren des Hohlraumes 4 hin zusammenlaufend ausgebildet.

Fig. 5 zeigt eine Schutzhülle 16; die zum Aufsetzen auf-den Grundkörper 1 nach den Figuren 1 bis 4 ausgebildet ist und zusammen mit diesem den erfindungsgemäßen Ampullenhalter 2 bildet.

Die Schutzhülle 16 besitzt einen hohlzylindrischen Aufbau, wobei der Innendurchmesser der Schutzhülle 16 geringfügig größer ist als der Außendurchmesser des Grundkörpers 1, so dass die Schutzhülle 16 mit ihrem proximalen Ende 17 über das distale Ende 18 des Grundkörpers 1 aufschiebbar ist.

An der Innenseite der Schutzhülle 16 sind, entsprechend den Längsstegen 13, sich einander gegenüberliegende Längsnuten 20 ausgebildet, von denen in Fig. 5 nur eine gestrichelt dargestellt ist. Die zweite Längsnut 20 ist in Fig. 6 zu erkennen.

Im Bereich des proximalen Endes 17 der Schutzhülle 16 ist eine außen liegende Ringnut 21 vorgesehen, die zwischen zwei ringförmigen Ansätzen 22, 23 ausgebildet ist. Am Boden der Ringnut 21 ist jeweils im Bereich der Längsnuten 20 eine Öffnung 24 in der Schutzhülle 16 ausgebildet, durch die das Innere der Schutzhülle 16 von außen zugänglich ist.

Der ringförmige Ansatz 23 bildet zusammen mit weiteren ringförmigen Ansätzen 25 einen Angriffsabschnitt 26 der Schutzhülle 16. Dabei sind die Außenseiten der ringförmigen Ansätze 25 schräg in Richtung zu dem distalen Ende 27 der Schutzhülle 16 hin zusammenlaufend ausgebildet.

Fig. 7 zeigt eine gegenüber Fig. 5 um 90° um die Längsachse 3 gedrehte Darstellung der Schutzhülle 16 im Längsschnitt. Dabei sind insbesondere die beiden Öffnungen 24 zu erkennen, über die das Innere der Schutzhülle 16 von außen zugänglich ist.

In Fig. 8 ist in den Hohlraum 4 des Grundkörpers 1 eine handelsübliche Zylinderampulle 28 eingesetzt. Die Zylinderampulle 28 besitzt einen zylindrischen Glaskörper 29, der mit Injektionsflüssigkeit 30 gefüllt ist.

An dem proximalen Ende 31 der Zylinderampulle 28 ist eine Öffnung 32 ausgebildet, in die ein Kolben 33 aus einem elastischen Material, beispielsweise aus Gummi, abdichtend eingesetzt ist. Der Kolben 33 besitzt an seinem proximalen Ende eine in Fig. 8 nicht dargestellte Öffnung, in die das freie Ende einer Kolbenstange 34 einsetzbar ist. Dabei besitzt das freie Ende der Kolbenstange 34 Koppelungselemente, mit denen eine feste Verkoppelung zwischen der Kolbenstange 34 und dem Kolben 33 möglich ist, so dass der Kolben 33 über die Kolbenstange 34 in Längsrichtung verschiebbar ist.

An dem distalen Ende 35 der Zylinderampulle 28 ist eine Abschlusskappe 36 vorgesehen, an der eine elastische Abschlussplatte, beispielsweise in Form einer Gummimembran 37, befestigt ist. Durch diese Gummimembran wird eine am distalen Ende 35 der Zylinderampulle 38 ausgebildete Austrittsöffnung 38 verschlossen, wobei die Gummimembran 37 nach Aufsetzen einer Injektionsnadel 39 (siehe Figuren 11 bis 14) durchstochen wird, wie es weiter unten näher beschrieben wird.

Aus Fig. 8 ist zu erkennen, dass bei einem Einsetzen der Zylinderampulle 28 in den Hohlraum 4 des Grundkörpers 1 die Gummimembran 37 an dem Ansatz 15 zur Anlage kommt und sich bei einem vollständigen Einschieben der Zylinderampulle 28 nach innen verformt, wie es in Fig. 8 dargestellt ist. Die mit dieser Verformung einhergehende Zwangsaspiration wird ebenfalls weiter unten näher beschrieben.

In Fig. 9 ist dargestellt, wie die Schutzhülle 16 vollständig über den Grundkörper 1 aufgeschoben ist. Während der ringförmige Ansatz 11 zum einen eine Fixierung des erfindungsgemäß ausgebildeten Ampullenhalters 2 an einem handelsüblichen Griffstück einer Injektionsspritze ermöglicht, ist aus Fig. 9 zu erkennen, dass der Ansatz 11 gleichzeitig als Anschlag für das proximale Ende 17 der Schutzhülle 16 dient.

Weiterhin ist zu erkennen, dass die Schutzhülle 16 und der Grundkörper 1 so ausgebildet sind, dass die Koppelungsvorrichtung 6 für die Injektionsnadel 39 bei vollständig aufgeschobener Schutzhülle 16 aus deren distalen Ende 27 herausragt. Dadurch ist gewährleistet, dass bei vollständig zurückgezogener Schutzhülle 16 auch die Injektionsnadel 39 über ihre gesamte Länge frei liegt.

Weiterhin ist in Fig. 9 und insbesondere deutlicher in Fig. 10 ein Rastelement 40 zu erkennen, das einen C-förmigen Basisteil 41 und zwei radial innen liegende Rastnasen 42 umfasst. Das Rastelement 40 ist in der Ringnut 21 der Schutzhülle 16 so angeordnet, dass sich die Rastnasen 42 durch die Öffnungen 24 der Schutzhülle 16 hindurch in das Innere der Schutzhülle 16 hinein erstrecken.

In den Rastnasen 42 sind Führungsnuten 43 ausgebildet, die die Längsstege 13 umgreifen und somit ein Verdrehen des Rastelementes 40 und damit ein Verdrehen der Schutzhülle 16 gegenüber dem Grundkörper 1 verhindern. Auf diese Weise wird vermieden, dass bei einem Verdrehen die nach innen ragenden Rastnasen 42 in die seitlichen Öffnungen 8 des Grundkörpers 1 eindringen und ein vollständiges Verschieben der Schutzhülle 16 auf dem Grundkörper 1 blockieren würden.

Im Folgenden wird anhand der Figuren 11 bis 14 die Verwendung eines erfindungsgemäß ausgebildeten Ampullenhalters 2 näher beschrieben.

Zur Vorbereitung der Injektion wird zunächst der als Einweg-Produkt aus Kunststoff hergestellte Ampullenhalter 2 aus seiner sterilen Verpackung entnommen. Die Schutzhülle 16 befindet sich dabei in ihrer vollständig zurückgezogenen Stellung auf dem Grundkörper 1, die im Folgenden als Ruheposition der Schutzhülle 16 bezeichnet wird. In dieser Ruheposition greifen die Rastnasen 42 in die an der Außenseite des Grundkörpers 1 vorgesehene Ringnut 14 ein, da das Rastelement 40 unter Vorspannung in der Ringnut 21 der Schutzhülle 16 angeordnet ist.

Die Ringnut 14 besitzt nur eine relativ geringe Tiefe, so dass eine Selbstentkopplung der Rastnasen 42 und der Ringnut 14 verhindert, eine gezielte Entkopplung durch Vorschieben der Schutzhülle 16 jedoch möglich ist.

Durch Anfassen der Schutzhülle 16 an dem Angriffsabschnitt 26 und gezieltes Vorschieben lässt sich die zwischen der distalseitig gelegenen Schulter der Ringnut 14 und den Rastnasen 42 wirkende Blockierung überwinden, so dass ein Verschieben der Schutzhülle 16 in distaler Richtung möglich ist, wie es weiter unten näher beschrieben wird. Diese Verschiebungsmöglichkeit kann durch eine entsprechende Ausgestaltung der Ringnut 14 sowie der Rastnasen 42, beispielsweise durch entsprechende Auflaufschrägen, weiter verbessert werden.

Nach Entnahme des Ampullenhalters 2 aus seiner sterilen Verpackung wird eine Zylinderampulle 28 über die Eintrittsöffnung 9 in den Hohlraum 4 des Grundkörpers 1 hinein geschoben, bis die Gummimembran 37 an dem Ansatz 15 zur Anlage kommt.

Anschließend wird der Ampullenhalter 2 mit seinem proximal gelegenen Ansatz 11 in eine Aufnahme 44 eines Griffstückes 45 einer handelsüblichen Injektionsspritze 46 eingesetzt. Das Griffstück 45 umfasst dabei eine längsverschiebbare Kolbenstange 34, an deren proximalen Ende ein Handgriff 47 und an deren distalen Ende ein nicht dargestellter, handelsüblicher Klemmabschnitt vorgesehen sind. Der Klemmabschnitt kann beispielsweise Spreizklauen umfassen, die durch Drehen eines am Handgriff 47 vorgesehenen Betätigungsabschnitts 48 radial ein- bzw. ausgefahren werden können.

Nach Fixieren des Ampullenhalters 2 an dem Griffstück 45 wird die Kolbenstange 34 soweit in distaler Richtung nach vorne geschoben, bis die Spreizklauen in eine proximale Öffnung des Kolbens 33 der Zylinderampulle 28 eintreten. Durch Verdrehen des Betätigungsabschnittes 48 werden die Spreizklauen radial nach außen bewegt, so dass eine sichere Verbindung zwischen der Kolbenstange 34 und dem Kolben 33 entsteht.

Anschließend wird die ebenfalls steril verpackte und als Einmalprodukt ausgebildete Injektionskanüle 49 aus ihrer sterilen Verpackung entnommen und auf die Koppelungsvorrichtung 6 des Ampullenhalters 2 aufgesetzt, beispielsweise mit dieser verschraubt. Die Injektionskanüle 49 umfasst eine distal gelegene Injektionsnadel 39, mit der die eigentliche Injektion erfolgt. An dem proximalen Ende der Injektionsnadel 39 ist ein üblicherweise aus Kunststoff bestehendes Koppelungselement 50, meist in Form eines Konus, vorgesehen, über den die Injektionsnadel 39 mit der Koppelungsvorrichtung 6 verkoppelbar ist. An dem proximalen Ende des Kopplungselements 50 ist eine Verlängerung der Injektionsnadel 39 in Form eines rückwärtigen Kanülenabschnitts 51 ausgebildet, der gemäß einem Pfeil 52 durch die Austrittsöffnung 5 hindurch in den Hohlraum 4 des Grundkörpers 1 einsetzbar ist.

Um Verletzungen zu vermeiden, ist die Injektionsnadel 39 in verpacktem Zustand üblicherweise von einer distalen Schutzkappe 7 vollständig abgedeckt, die in Fig. 11 teilweise aufgebrochen dargestellt ist. Diese Schutzkappe 7 wird erst nach erfolgtem Befestigen der Injektionskanüle 49 an der Kopplungsvorrichtung 6 abgezogen. Auch der rückwärtige Kanülenabschnitt 51 ist in verpacktem Zustand in ähnlicher Weise durch eine nicht dargestellte proximale Schutzkappe abgedeckt. Diese muss selbstverständlich vor dem Befestigen der Injektionskanüle 49 entfernt werden.

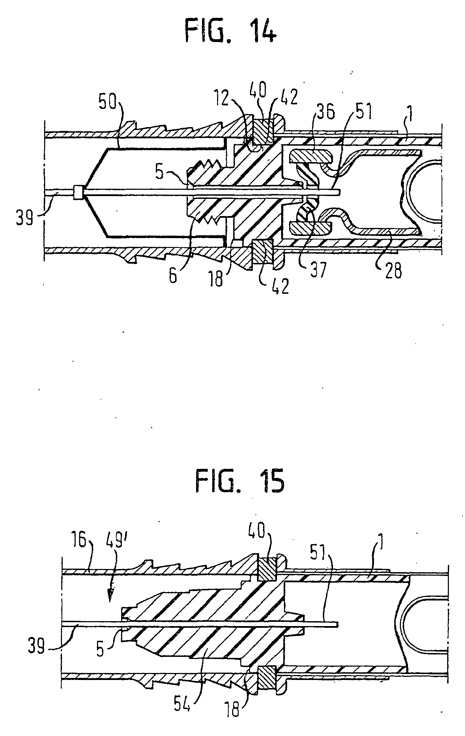

Wie aus Fig. 14 zu erkennen ist, durchsticht der rückwärtige Kanülenabschnitt 51 die Gummimembran 37, so dass die Injektionsflüssigkeit 30 durch den rückwärtigen Kanülenabschnitt 51 zu der Injektionsnadel 39 geführt werden kann.

Nachdem die Injektionsnadel 39 vollständig auf dem Grundkörper 1 aufgesetzt ist, wird die Schutzkappe 7 entfernt, so dass die Injektionsspritze gebrauchsfertig ist, wie es in Fig. 12 dargestellt ist. Die Schutzhülle 16 befindet sich dabei weiter in ihrer Ruheposition.

Nach erfolgter Punktierung wird die Kolbenstange 34 über den Handgriff 47 leicht in distaler Richtung verschoben, bis die Abschlusskappe 36 der Zylinderampulle 28 an der distalen Stirnseite des Hohlraumes 4 zur Anlage kommt. In dieser Stellung wird die Gummimembran 37 durch den Ansatz 15 nach innen gedrückt, wie es in Fig. 8 dargestellt ist.

Anschließend wird das Vorschieben der Kolbenstange 34 gestoppt und der Handgriff 47 freigegeben, so dass aufgrund der Elastizität der Gummimembran 37 die Zylinderampulle 28 und damit der Handgriff 47 wieder leicht in proximaler Richtung verschoben werden. Durch diese Verschiebung entsteht innerhalb der Zylinderampulle 28 ein Unterdruck, so dass eine automatische Aspiration, d. h. eine Zwangsaspiration erfolgt, ohne dass der Handgriff 47 aktiv zurückgezogen werden muss.

Tritt bei dieser Zwangsaspiration ein Blutfaden in die Zylinderampulle 28 ein, der über die seitlichen Öffnungen 8 beobachtete werden kann, so muss der Punktionsvorgang wiederholt werden.

Die Injektion erfolgt in bekannter Weise, wobei sich die Schutzhülle 16 während der gesamten Injektion in der in Fig. 11 gezeigten Ruheposition befindet, in der sie durch das in die Ringnut 14 eingreifende Rastelement 40 gegenüber einem versehentlichen Vorschieben gesichert ist.

Nach erfolgter Injektion wird die Injektionsnadel 39 in üblicher Weise wieder aus dem Gewebe herausgezogen, die Injektionsspritze wird jedoch nicht wie bei bekannten Systemen mit ungesicherter Injektionsnadel 39 an die Arzthelferin übergeben. Gemäß der Erfindung wird unmittelbar nach der Injektion die Schutzhülle 16 vom Arzt gemäß einem Pfeil 53 in Fig. 13 nach vorne in distaler Richtung verschoben, bis sich die Injektionsnadel 39 vollständig innerhalb der Schutzhülle befindet und die Spitze der Injektionsnadel 39 von dem distalen Ende der Schutzhülle 16 überragt wird.

Während des Vorschiebens wird die Schutzhülle unverdrehbar auf dem Grundkörper 1 geführt, bis die Rastnasen 42 des Rastelementes 40 aufgrund der in radialer Richtung wirkenden Vorspannung des Rastelementes 40 in die am distalen Ende des Grundkörpers 1 ausgebildete Ringnut 12 eintreten. Aufgrund der Tiefe der Ringnut 12 entsteht eine nicht lösbare Verrastung zwischen der Schutzhülle 16 und dem Grundkörper 1, so dass ein versehentliches Zurückziehen der Schutzhülle 16 zuverlässig verhindert wird. Dieser in Fig. 13 dargestellte Zustand der Schutzhülle 16 wird als Sicherungsposition bezeichnet.

In der nun gesicherten Stellung kann die Injektionsspritze ohne jegliche Gefahr an die Arzthelferin übergeben werden, welche, ebenfalls ohne Verletzungsrisiko, den Ampullenhalter 2 zusammen mit der gesicherten Injektionsnadel 39 aus dem Handgriff 47 entnehmen und zur Entsorgung ablegen kann.

In Fig. 14 ist in einem Teilschnitt dargestellt, wie die beiden Rastnasen 42 vollständig in die Ringnut 12 eingreifen, wodurch ein versehentliches Zurückschieben der Schutzhülle 16 zuverlässig verhindert wird.

In der in Fig. 15 dargestellten Ausführungsform ist die Injektionskanüle 49' in den Grundkörper 1 des Ampullenhalters integriert. Der Grundkörper 1 besitzt dazu an seinem distalen Ende 18 anstelle der Kopplungsvorrichtung 6 einen einstückig mit dem Grundkörper 1 ausgebildeten, konusförmigen Ansatz 54, durch den sich die Austrittsöffnung 5 hindurch erstreckt. Die Injektionskanüle 49' ist in der Austrittsöffnung 5 fest verankert, beispielsweise verklebt oder eingespritzt, und bildet somit einen integralen Bestandteil des Grundkörpers 1.

Die Verwendung dieser Ausführung erfolgt in gleicher Weise wie bereits zu den Fig. 1 bis 14 beschrieben. Lediglich das Aufschrauben der Injektionskanüle 49' entfällt bei der in Fig. 15 dargestellten Variante, so dass mit dieser Ausführungsform ein Schritt bei der Vorbereitung der Injektion eingespart werden kann.

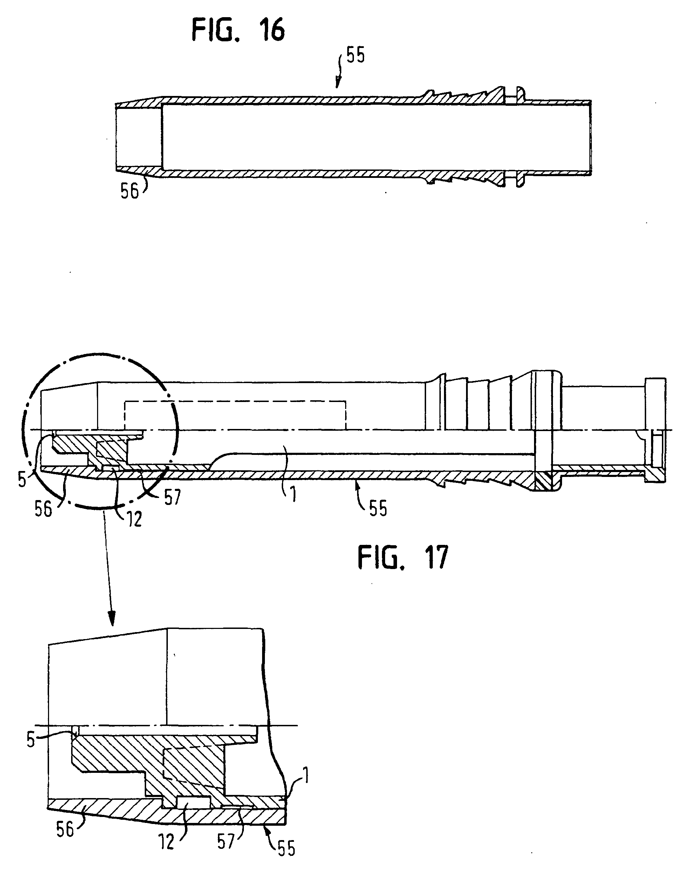

Fig. 16 zeigt eine weitere Schutzhülle 55, die sich von der Schutzhülle 16 dadurch unterscheidet, dass an ihrem distalen Ende ein Verlängerungsabschnitt 56 vorgesehen ist, der, wie in Fig. 17 zu sehen ist, über die Austrittsöffnung 5 hinausragt, wenn sich die Schutzhülle 55 in der Ruheposition befindet. Durch den Verlängerungsabschnitt 56 wird verhindert, dass die Austrittsöffnung 5, beispielsweise beim Entnehmen des Ampullenhalters aus seiner sterilen Verpackung, berührt werden kann, wodurch die Austrittsöffnung 5 kontaminiert werden könnte. Denn aufgrund einer kontaminierten Austrittsöffnung 5 könnten Partikel in die zu injizierende Flüssigkeit gelangen und den Patienten infizieren.

In Fig. 17 ist ein Teillängsschnitt einer Ausführungsform des Ampullenhaltes dargestellt, die sich von den vorher beschriebenen Ausführungsformen der Fig. 1 bis 15 dadurch unterscheidet, dass der Grundkörper 1 eine weitere Ringnut 57 aufweist, in die die Rastnasen 42 der Schutzhülle 55 eingreifen können. Die Ringnut 57 befindet sich auf dem Grundkörper 1. proximal von der Ringnut 12 und distal von der Ringnut 14, wobei die Ringnut 57 in der Nähe der Ringnut 14 angeordnet ist. Greifen die Rastnasen 42 in die Ringnut 57, befindet sich die Schutzhülle 55 in einer Zwischensicherungsposition, in der die Schutzhülle 55 die Injektionsnadel 39 vollständig abdeckt.

Die Ringnut 57 weist eine vergleichsweise geringe Tiefe auf, so dass eine Selbstentkopplung der Rastnasen 42 und der Ringnut 57 verhindert werden kann, eine gezielte Entkopplung durch Verschieben der Schutzhülle 55 jedoch möglich ist. Die Verschiebungsmöglichkeit der Ringnut 57 sowie der Rastnasen 42 kann beispielsweise durch entsprechende Auflaufschrägen verbessert werden.

Somit kann die Schutzhülle 55 aus einer Ruheposition in die Zwischensicherungsposition geschoben werden, um beispielsweise während einer Behandlung eine leere Ampulle aus dem Ampullenhalter zu entnehmen und gegen eine volle auszutauschen, ohne dass die Gefahr von Nadelstichen einerseits und eines ungewollten Übergangs in die Sicherungsposition andererseits besteht. Nach dem Wechsel der Ampulle wird die Schutzhülle zurück in die Ruheposition geschoben und die Behandlung fortgesetzt. Nach Beendigung der Behandlung kann die Schutzhülle über die Zwischensicherungsposition in die unlösbare Sicherungsposition geschoben werden.

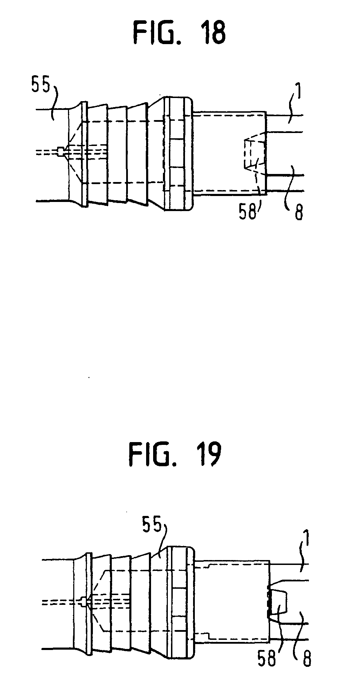

Fig. 18 zeigt den Grundkörper 1 und die Schutzhülle 55, wobei sich die Schutzhülle 55 in der Zwischensicherungsposition befindet. Am distalen Ende der seitlichen Öffnung 8 des Grundkörpers 1 ist eine Markierung 58 angebracht. Die Markierung 58 ist ein insbesondere trapezförmig ausgebildeter Ansatz, der zum proximalen Ende der seitlichen Öffnung 8 des Grundkörpers 1 weist. In der Zwischensicherungsposition ist die Markierung 58 von der Schutzhülle 55 vollständig abgedeckt, wobei die proximalen Endkanten der Markierung 58 und der Schutzhülle 55 bündig zueinander ausgerichtet sind.

In Fig. 19 ist die in Fig. 18 dargestellte Schutzhülle 55 in der Sicherungsposition gezeigt. Wenn sich die Schutzhülle 55 in der Sicherungsposition befindet, ragt die Markierung 58 über die Schutzhülle 55 hinaus.

Mit Hilfe der Markierung 58 kann der Arzt somit optisch sofort erkennen, in welcher Position sich die Schutzhülle 55 bezüglich des Grundkörpers 1 befindet, d.h. ob sich die Schutzhülle 55 in der Zwischensicherungsposition oder in der Sicherungsposition befindet.

Das Risiko einer Stichverletzung nach erfolgter Injektion ist mit der Erfindung praktisch ausgeschlossen. Dabei setzt die Wirkung der Erfindung unmittelbar nach Beendigung der Injektion ein, so dass insbesondere der gefährliche Zeitpunkt der Übergabe der Injektionsspritze vom Arzt zur Arzthelferin durch die Erfindung risikofrei gehalten wird. Bei einer Weiterbildung der Erfindung ist sogar ein zusätzlicher Schutz vor Stichverletzungen während einer Behandlung gegeben.

### Bezugszeichenliste

- 1: Grundkörper
- 2: Ampullenhalter
- 3: Längsachse
- 4: Hohlraum
- 5: Austrittsöffnung
- 6: Koppelungsvorrichtung
- 7: Schutzkappe
- 8: seitliche Öffnungen
- 9: Eintrittsöffnungen
- 10: Zylinderampulle
- 11: ringförmiger Ansatz
- 12: Ringnut
- 13: Längsstege
- 14: Ringnut
- 15: Ansatz
- 16: Schutzhülle
- 17: proximales Ende der Schutzhülle
- 18: distales Ende des Grundkörpers
- 19: proximales Ende des Grundkörpers
- 20: Längsnuten
- 21: Ringnut
- 22: ringförmiger Ansatz
- 23: ringförmiger Ansatz
- 24: Öffnung
- 25: ringförmige Ansätze
- 26: Angriffsabschnitt
- 27: distales Ende der Schutzhülle
- 28: Zylinderampulle
- 29: Glaskörper
- 30: Injektionsflüssigkeit
- 31: proximales Ende der Zylinderampulle
- 32: Öffnung
- 33: Kolben
- 34: Kolbenstange
- 35: distales Ende der Zylinderampulle
- 36: Abschlusskappe
- 37: Gummimembran
- 38: Austrittsöffnung
- 39: Injektionsnadel
- 40: Rastelement
- 41: C-förmiger Basisteil
- 42: Rastnasen
- 43: Führungsnuten
- 44: Aufnahme
- 45: Griffstück
- 46: Injektionsspritze
- 47: Handgriff
- 48: Betätigungsabschnitt
- 49: Injektionskanüle
- 50: Koppelungselement
- 51: rückwärtiger Kanülenabschnitt
- 52: Pfeil
- 53: Pfeil
- 54: Ansatz
- 55: Schutzhülle
- 56: Verlängerungsabschnitt
- 57: Ringnut
- 58: Markierung

## Patentansprüche

1. Ampullenhalter für Zylinderampullenspritzen mit einem einen innen liegenden Hohlraum (4) aufweisenden Grundkörper (1), in den eine eine zu injizierende Flüssigkeit (30) enthaltende Ampulle (28) einsetzbar ist, wobei am proximalen Ende (19) des Grundkörpers (1) eine Eintrittsöffnung (9) ausgebildet ist, über die eine Kolbenstange (34) der Zylinderampullenspritze in den Hohlraum (4) einschiebbar ist, am distalen Ende (18) des Grundkörpers (1) eine mit einer Injektionsnadel (39) koppelbare oder versehene Austrittsöffnung (5) ausgebildet ist, und die zu injizierende Flüssigkeit (30) bei einem Verschieben der Kolbenstange (34) aus der Ampulle (28) durch die Austrittsöffnung (5) oder die Injektionsnadel (39) herauspressbar ist,
**dadurch gekennzeichnet,**
**dass** an der Außenseite des Grundkörpers (1) eine Schutzhülle (16, 55) vorgesehen ist, die von einer zurückgezogenen Ruheposition, in der die Injektionsnadel (39) im Wesentlichen über ihre gesamte Länge frei liegt, in eine vorgeschobene Sicherungsposition verschiebbar ist, in der die Injektionsnadel (39) über ihre gesamte Länge innerhalb der Schutzhülle angeordnet ist.

2. Ampullenhalter nach Anspruch 1,
**dadurch gekennzeichnet,**
**dass** die Injektionsnadel (39) zumindest im Bereich ihrer Spitze bei in der Sicherungsposition befindlicher Schutzhülle (16, 55) von dieser seitlich vollständig umschlossen ist.

3. Ampullenhalter nach einem der vorhergehenden Ansprüche,
**dadurch gekennzeichnet,**
**dass** die Injektionsnadel (39) über ihre gesamte Länge bei in der Sicherungsposition befindlicher Schutzhülle (16, 55) von dieser seitlich vollständig umschlossen ist.

4. Ampullenhalter nach einem der vorhergehenden Ansprüche,
**dadurch gekennzeichnet,**
**dass** das distale Ende (27) der sich in der Sicherungsposition befindenden Schutzhülle (16, 55) über die Spitze der Injektionsnadel (39) hinausragt.

5. Ampullenhalter nach einem der vorhergehenden Ansprüche,
**dadurch gekennzeichnet,**
**dass** die Schutzhülle (16, 55) in der Sicherungsposition gegenüber dem Grundkörper (1) unverschiebbar fixierbar ist.

6. Ampullenhalter nach Anspruch 5,
**dadurch gekennzeichnet,**
**dass** bei Erreichen der Sicherungsposition eine automatische Fixierung der Schutzhülle (16, 55) erfolgt.

7. Ampullenhalter nach einem der Ansprüche 5 oder 6,
**dadurch gekennzeichnet,**
**dass** zur Fixierung der Schutzhülle (16, 55) an dieser zumindest ein Rastelement (40) vorgesehen ist, das in der Sicherungsposition mit zumindest einem an dem Grundkörper (1) vorgesehenen ersten Gegenelement (12) zur Fixierung der Schutzhülle (16, 55) zusammenwirkt.

8. Ampullenhalter nach Anspruch 7,
**dadurch gekennzeichnet,**
**dass** das Rastelement (40) als separates Teil ausgebildet ist.

9. Ampullenhalter nach Anspruch 7,
**dadurch gekennzeichnet,**
**dass** das Rastelement einstückig mit der Schutzhülle ausgebildet ist.

10. Ampullenhalter nach einem der Ansprüche 7, 8 oder 9,
**dadurch gekennzeichnet,**
**dass** das Rastelement (40) zumindest eine Rastnase (42) umfasst und dass das erste Gegenelement als Rastausnehmung (12) ausgebildet ist, in die die Rastnase (42) in der Sicherungsposition eingreift.

11. Ampullenhalter nach einem der Ansprüche 7 bis 10,
**dadurch gekennzeichnet,**
**dass** das Rastelement (40) im Bereich des proximalen Endes (17) der Schutzhülle (16, 55) ausgebildet ist.

12. Ampullenhalter nach einem der Ansprüche 7 bis 11,
**dadurch gekennzeichnet,**
**dass** das erste Gegenelement (12) im Bereich des distalen Endes (18) des Grundkörpers (1) ausgebildet ist.

13. Ampullenhalter nach einem Ansprüche 7 bis 12,
**dadurch gekennzeichnet,**
**dass** das Rastelement (40) einen C-förmigen Basisteil (41) umfasst, an dessen radialer Innenseite eine oder mehrere, bevorzugt zwei sich radial nach innen erstreckende Rastnasen (42) ausgebildet sind.

14. Ampullenhalter nach Anspruch 13,
**dadurch gekennzeichnet,**
**dass** zwei Rastnasen vorgesehen sind, die sich einander gegenüberliegend angeordnet sind.

15. Ampullenhalter nach Anspruch 13 oder 14,
**dadurch gekennzeichnet,**
**dass** die Schutzhülle (16, 55) von dem C-förmigen Basisteil (41) radial umgriffen wird.

16. Ampullenhalter nach einem der Ansprüche 13, 14 oder 15,
**dadurch gekennzeichnet,**
**dass** in der außen liegenden Oberfläche der Schutzhülle (16, 55) eine ringförmige Vertiefung (21), insbesondere eine Ringnut vorgesehen ist, in der der C-förmige Basisteil (41) angeordnet ist.

17. Ampullenhalter nach einem der Ansprüche 13 bis 16,
**dadurch gekennzeichnet,**
**dass** in der Schutzhülle (16, 55) für jede Rastnase (42) eine Öffnung (24) vorgesehen ist, durch die sich die jeweilige Rastnase (42) hindurch in Richtung zur Außenseite des Grundkörpers (1) hin erstreckt.

18. Ampullenhalter nach einem der Ansprüche 7 bis 17,
**dadurch gekennzeichnet,**
**dass** die Rastelemente (40) unter Vorspannung an der Außenseite des Grundkörpers (1) anliegen.

19. Ampullenhalter nach einem der vorhergehenden Ansprüche,
**dadurch gekennzeichnet,**
**dass** an der Außenseite des Grundkörpers (1) eine oder mehrere Längsführungen (13) für die Schutzhülle (16, 55) vorgesehen sind.

20. Ampullenhalter nach Anspruch 19,
**dadurch gekennzeichnet,**
**dass** die Längsführungen als Längsstege (13) ausgebildet sind.

21. Ampullenhalter nach Anspruch 20,
**dadurch gekennzeichnet,**
**dass** in den Rastelementen (40) Führungsnuten (43) ausgebildet sind, die beim Verschieben der Schutzhülle (16, 55) auf den Längsstegen (13) geführt werden.

22. Ampullenhalter nach Anspruch 19,
**dadurch gekennzeichnet,**
**dass** die Längsführungen als Längsnuten ausgebildet sind.

23. Ampullenhalter nach Anspruch 22,
**dadurch gekennzeichnet**
**dass** die Rastelemente beim Verschieben der Schutzhülle in den Längsnuten geführt werden.

24. Ampullenhalter nach einem der vorhergehenden Ansprüche,
**dadurch gekennzeichnet,**
**dass** die Schutzhülle und der Grundkörper (1) gegeneinander unverdrehbar ausgebildet sind.

25. Ampullenhalter nach einem der vorhergehenden Ansprüche,
**dadurch gekennzeichnet,**
**dass** die Schutzhülle (16, 55) in der Ruheposition mit dem Grundkörper (1) unverschiebbar gekoppelt ist, wobei diese Kopplung lösbar ausgebildet ist.

26. Ampullenhalter nach einem der vorhergehenden Ansprüche,
**dadurch gekennzeichnet,**
**dass** die Schutzhülle (16, 55) in der Ruheposition mit dem Grundkörper (1) lösbar verrastet ist.

27. Ampullenhalter nach einem der vorhergehenden Ansprüche,
**dadurch gekennzeichnet,**
**dass** die lösbare Verrastung in der Ruheposition über das Rastelement (40) und ein im proximalen Bereich (19) des Grundkörpers (1) vorgesehenes zweites Gegenelement (14), insbesondere eine Ringnut, erfolgt.

28. Ampullenhalter nach einem der vorhergehenden Ansprüche,
**dadurch gekennzeichnet,**
**dass** das distale Ende (27) der Schutzhülle (55) über das distale Ende (18) des Grundkörpers (1), insbesondere über die Austrittsöffnung (5), hinausragt, wenn sich die Schutzhülle (55) in der Ruheposition befindet.

29. Ampullenhalter nach einem der vorhergehenden Ansprüche,
**dadurch gekennzeichnet,**
**dass** die Schutzhülle (16, 55) im Wesentlichen zylinderförmig ausgebildet ist.

30. Ampullenhalter nach einem der vorhergehenden Ansprüche,
**dadurch gekennzeichnet,**
**dass** der Grundkörper (1) zumindest bereichsweise zylinderförmig ausgebildet ist.

31. Ampullenhalter nach einem der vorhergehenden Ansprüche,
**dadurch gekennzeichnet,**
**dass** der Grundkörper (1) eine oder mehrere, bevorzugt zwei seitliche Öffnungen (8) aufweist, durch die der innen liegende Hohlraum (4) von außen zugänglich ist.

32. Ampullenhalter nach Anspruch 31,
**dadurch gekennzeichnet,**
**dass** sich die eine oder mehrere Öffnungen (8) in Richtung der Längsachse (3) des Grundkörpers (1) erstrecken.

33. Ampullenhalter nach einem der vorhergehenden Ansprüche,
**dadurch gekennzeichnet,**
**dass** sich im Inneren des Grundkörpers (1) ausgehend von der Austrittsöffnung ein Ansatz (15) in den Hohlraum (4) hinein erstreckt.

34. Ampullenhalter nach Anspruch 33,
**dadurch gekennzeichnet,**
**dass** das sich zum Inneren des Hohlraums (4) hin erstreckende freie Ende des Ansatzes (15) eine geringere Außenabmessung, insbesondere einen geringeren Außendurchmesser besitzt als eine am distalen Ende einer in den Hohlraum (4) einzusetzenden Ampulle (28) vorgesehene, üblicherweise mit einer Membran (37) verschlossene Öffnung.

35. Ampullenhalter nach Anspruch 34,
**dadurch gekennzeichnet,**
**dass** der Ansatz (15) so angeordnet ist, dass bei vollständig in den Hohlraum (4) eingesetzter Ampulle (28) und Vorschieben der Kolbenstange (34) die Membran (37) durch den Ansatz (15) zum Inneren der Ampulle (28) hin gedrückt wird.

36. Ampullenhalter nach einem der Ansprüche 33 bis 35,
**dadurch gekennzeichnet,**
**dass** der Ansatz (15) ringförmig ausgebildet ist und die Austrittsöffnung (5) ringförmig, insbesondere konzentrisch umschließt.

37. Ampullenhalter nach einem der vorhergehenden Ansprüche,
**dadurch gekennzeichnet,**
**dass** der Grundkörper (1) und/ oder die Schutzhülle (16, 55) aus Kunststoff bestehen.

38. Ampullenhalter nach einem der vorhergehenden Ansprüche,
**dadurch gekennzeichnet,**
**dass** der Grundkörper (1) und/oder die Schutzhülle (16, 55) aus transparentem oder durchsichtigem Material bestehen.

39. Ampullenhalter nach einem der vorhergehenden Ansprüche,
**dadurch gekennzeichnet,**
**dass** der Grundkörper (1) und die Schutzhülle (16, 55) als Einmalprodukte ausgebildet sind.

40. Ampullenhalter nach einem der vorhergehenden Ansprüche,
**dadurch gekennzeichnet,**
**dass** die Injektionsnadel (39) als vom Grundkörper (1) getrenntes Teil ausgebildet ist, das mit der Austrittsöffnung (5) koppelbar ist.

41. Ampullenhalter nach Anspruch 40,
**dadurch gekennzeichnet,**
**dass** im Bereich des proximalen Endes der Injektionsnadel (39) ein Kopplungselement (50) ausgebildet ist, über das die Injektionsnadel (39) an der Austrittsöffnung (5) ankoppelbar ist.

42. Ampullenhalter nach einem der Ansprüche 1 bis 39,
**dadurch gekennzeichnet,**
**dass** die Injektionsnadel (39) in den Grundkörper (1) integriert, insbesondere mit dem Grundkörper verspritzt oder verklebt ist.

43. Ampullenhalter nach Anspruch 42,
**dadurch gekennzeichnet,**
**dass** zur Aufnahme der Injektionsnadel (39) im Bereich des distalen Endes (18) des Grundkörpers (1) ein insbesondere konusförmiger Ansatz (53) ausgebildet ist, durch den sich die Austrittsöffnung (5) hindurch erstreckt.

44. Ampullenhalter nach einem der vorhergehenden Ansprüche,
**dadurch gekennzeichnet,**
**dass** zwischen der Ruheposition und der Sicherungsposition der Schutzhülle (55) eine Zwischensicherungsposition vorgesehen ist, in der die Schutzhülle gegen Verschieben gesichert ist, wobei diese Sicherung lösbar ausgebildet ist.

45. Ampullenhalter nach Anspruch 44,
**dadurch gekennzeichnet,**
**dass** die Injektionsnadel (39) über ihre gesamte Länge innerhalb der Schutzhülle (55) angeordnet ist, wenn sich diese in der Zwischensicherungsposition befindet.

46. Ampullenhalter nach Anspruch 44 oder 45,
**dadurch gekennzeichnet,**
**dass** die Schutzhülle (55) in der Zwischensicherungsposition mit dem Grundkörper (1) lösbar verrastbar ist.

47. Ampullenhalter nach Anspruch 46,
**dadurch gekennzeichnet,**
**dass** die lösbare Verrastung in der Zwischensicherungsposition über das Rastelement (40) und ein im distalen Bereich des Grundkörpers (1) vorgesehenes drittes Gegenelement (57), insbesondere eine Ringnut, erfolgt.

48. Ampullenhalter nach einem der Ansprüche 44 bis 47,
**dadurch gekennzeichnet,**
**dass** am Grundkörper (1) und/oder an der Schutzhülle (55) eine Markierung (58) zum Anzeigen der Sicherungsposition bzw. der Zwischensicherungsposition vorgesehen ist.

49. Ampullenhalter nach Anspruch 48,
**dadurch gekennzeichnet,**
**dass** die Markierung (58) am distalen Ende der seitlichen Öffnung (8) des Grundkörpers (1) angebracht ist.
